# EUROPÄISCHE PATENTSCHRIFT

(11) **EP 2 570 100 B1**
(45) Veröffentlichungstag und Bekanntmachung des Hinweises auf die Patenterteilung: **26.02.2020**
(21) Anmeldenummer: 12188573.5
(22) Anmeldetag: 05.03.2004
(51) Int. Cl.: A61C 13/00, A61C 13/097

(54) **Vorrichtung zur Auswahl eines Bereichs eines in einer 3D-Darstellung dargestellten dentalen Restaurationskörpers und Verfahren dazu**
Device for selecting an area of a dental restoration body, which is depicted in a 3D representation, and method therefor
Dispositif permettant de sélectionner une zone d'un corps de restauration dentaire figurant sur une représentation tridimensionnelle, et procédé correspondant

(30) Priorität: 05.03.2003 DE 10309839
(43) Veröffentlichungstag der Anmeldung: 20.03.2013
(62) Teilanmeldung aus: 04717552.6
(73) Patentinhaber: Sirona Dental Systems GmbH, 64625 Bensheim (DE)
(72) Erfinder: Hanisch, Heinrich, 64846 Gross-Zimmern (DE); Orth, Ulrich, 64646 Heppenheim (DE); Wedler, Volker, 69493 Hirschberg (DE)
(74) Vertreter: Özer, Alpdeniz

(56) Entgegenhaltungen:
- US-A- 5 273 429
- US-A- 6 049 743

## Beschreibung

### Technisches Gebiet

Die Erfindung betrifft eine Vorrichtung zur Auswahl eines Bereichs eines in einer 3D-Darstellung dargestellten dentalen Restaurationskörpers und ein Verfahren hierfür.

### Stand der Technik

Im Stand der Technik sind CAD/CAM-Systeme zur Konstruktion und Herstellung von Zahnrestaurationskörpern bekannt, die anhand einer Präparationsaufnahme eines präparierten Zahns in einer Zahndatenbank enthaltene Zähne in Form von charakteristischen Linienzügen wie Präparationsrand, Äquatorlinie, Randleiste, Fissur verwenden, um einen Restaurationskörper zu konstruieren und herzustellen.

Zur Verbesserung der Passgenauigkeit ist es weiterhin vorgesehen, eine in der Zahndatenbank vorhandene Okklusalfläche zumindest teilweise durch Kopieren auf den zu erstellenden Restaurationskörper zu übertragen.

In US 6,049,743 ist ein Verfahren zur Konstruktion eines Zahnmodells offenbart, bei dem das Zahnmodell in mehrere Bereiche aufgeteilt wird, die rechteckig geformt sein können oder durch den dentalspezifische Strukturen, wird Zahnhöcker, Zahnfurchen oder Zahnkanten, begrenzt sein können. Zur Verformung des Zahnmodells können die einzelnen Bereiche als Deformationseinheiten verwendet werden, wobei ein bestimmter Bereich verformt wird, ohne das die angrenzenden Bereiche verändert werden. Die Bereiche können auch durch Hilfslinien begrenzt werden, die beispielsweise Verlängerungen der natürlichen Zahnfurchen darstellen.

Für die gezielte Veränderung einer dentalen Konstruktion mit Hilfe eines CAD-Systems ist es stets notwendig, Bereiche, die mit bestimmten Konstruktionswerkzeugen bearbeitet werden sollen, auszuwählen.

Die Auswahl des Bereiches erfolgt im Stand der Technik dabei über ein Menü oder über einen Dialog. Darüber hinaus konnte die Bereichsfestlegung auch durch Einzeichnen einer Begrenzungslinie erfolgen.

Nachteilig ist hier, dass die Grenzen der zu verändernden Bereiche und damit die konstruierten Zahnformen nicht dentalspezifisch sind.

### Darstellung der Erfindung

Ein Gegenstand der Erfindung ist eine Vorrichtung zur Auswahl eines Bereichs eines in einer 3D-Darstellung dargestellten dentalen Restaurationskörpers, umfassend ein CAD-System mit einer Anzeigeeinheit zur Darstellung des Restaurationskörpers und mit einer Eingabeeinheit, über die ein auf der Anzeigeeinheit angezeigter Zeiger steuerbar ist, wobei zumindest Teile der Bereichsgrenze als dentalspezifische Linien ausgebildet sind. Die Vorrichtung ist so gestaltet, dass der Bereich durch Auswahl in der 3D-Darstellung angezeigter dentalspezifischer Punkte festlegbar ist und dass durch Auswahl eines dentalspezifischen Punktes für die Position der Höckerspitze der Bereich des entsprechenden Höckers bis zu seinen Höckergrenzen auswählbar ist und dass die untere Grenze des Bereichs durch die Äquatorlinie gebildet ist, wobei die Auswahl durch Anklicken des dentalspezifischen Punktes mittels der Eingabeeinheit über den Zeiger erfolgt.

Dies hat den Vorteil, dass eine einfache intuitive Bedienbarkeit bereitgestellt wird, indem unmittelbar in der zu bearbeitenden Darstellung gearbeitet wird. Der zu bearbeitende Bereich wird folglich intuitiv durch Auswahl einer Linie oder eines Punktes ausgewählt, die einem der dentaltypischen Bereich zugeordnet ist.

Weitere Ausgestaltungen der Erfindung sind in den Unteransprüchen wiedergegeben.

Als dentalspezifische Punkte kommen die Position einer oder mehrerer Höckerspitzen in Betracht, und zwar einzeln oder zu mehreren oder alle gemeinsam.

Vorteilhafterweise bestimmt der ausgewählte Teil der Linie den Teil des Zahnes, in der der Bereich liegt. Damit erfolgt die Auswahl unmittelbar anhand der Seitenorientierung in der 3D-Darstellung.

Vorteilhafterweise ist durch Auswahl eines dentalspezifischen Punktes für die Position der Höckerspitze der Bereich des entsprechenden Höckers bis zu seinen Höckergrenzen auswählbar, wobei die untere Grenze des Bereichs durch die Äquatorlinie gebildet sein kann.

Gemäß einer Weiterbildung ist der ausgewählte Bereich unterscheidbar dargestellt.

Gemäß einer Weiterbildung sind Auswerte- und/oder Vergleichsmittel für geometrische Daten des ausgewählten Bereichs vorgesehen. Damit ist es möglich, den ausgewählten Bereich in einem Speicher abzuspeichern oder mit einem in einem Speicher abgespeicherten Bereich einer anderen Restauration oder eines beliebigen anderen Zahns zu vergleichen.

Gemäß einer Weiterbildung ist der ausgewählte Bereich mit Hilfe eines Konstruktionswerkzeugs eines CAD-Systems bearbeitbar ist. Durch die Auswahl dentalspezifischer Punkte oder eines Präparationsrandes ist der mit dem Konstruktionswerkzeug zu bearbeitende Bereich festgelegt.

Vorteilhafterweise lässt das Werkzeug zur Veränderung des Bereiches die Bereichsgrenzen unverändert, wobei zu dem Punkt der stärksten Änderung hin ein stetiger Verlauf der Änderung vorgesehen ist. Dies ermöglicht ein gezieltes Bearbeiten des ausgewählten Bereichs unter Berücksichtigung der dentalspezifischen Gegebenheiten.

Vorteilhafterweise liegt der Punkt der stärksten Änderung auf einer dentalspezifischen Linie oder auf einem dentalspezifischen Punkt. Dadurch lässt sich sicherstellen, dass die Bearbeitung mit dem Konstruktionswerkzeug nicht unvorhergesehene Änderungen in Bereichen vornimmt, die außerhalb der Aufmerksamkeit des Bearbeiters liegen.

Ein weiterer Gegenstand der Erfindung ist ein Verfahren zur Auswahl eines Bereichs eines in einer 3D-Darstellung dargestellten dentalen Restaurationskörpers umfassend ein CAD-System mit einer Anzeigeeinheit zur Darstellung des Restaurationskörpers und mit einer Eingabeeinheit, über die ein auf der Anzeigeeinheit angezeigter Zeiger steuerbar ist, wobei dem ausgewählten Bereich Bereichsgrenzen zugeordnet werden, die zumindest zum Teil als dentalspezifische Linien ausgebildet sind, dadurch gekennzeichnet, dass durch Auswahl eines dentalspezifischen Punktes für die Position der Höckerspitze der Bereich der entsprechenden Höcker bis zu seinen Höckergrenzen ausgewählt wird und dass die untere Grenze des Bereichs durch die Äquatorlinie gebildet ist, wobei die Auswahl durch Anklicken des dentalspezifischen Punktes mittels der Eingabeeinheit über den Zeiger erfolgt.

Eine derartige Auswahl hat den Vorteil, dass für den Benutzer klar ist, welchen Bereich er gerade auswählt.

### Kurzbeschreibung der Zeichnung

Die erfindungsgemäße Vorrichtung wird anhand der Zeichnung erläutert. Es zeigt:
- Fig. 1: einen Restaurationskörper in Form eines Backenzahns aus mesialer Richtung gesehen mit einem der vorderen Hälfte des Zahns entsprechenden ausgewählten Bereich,
- Fig. 2: den Zahn aus Fig. 1 mit der rechten Hälfte des Zahn als ausgewählten Bereich, die
- Fig. 2a,b: den Zahn aus Fig. 2 mit der rechten Hälfte des Zahn als ausgewählten Bereich in einer Gegenüberstellung vor einer Veränderung (Fig. 2a) und nach einer Veränderung (Fig. 2b), die
- Fig. 3: den Zahn aus Fig. 1 in einer Schrägansicht mit einer Seitenfläche der vorderen Hälfte des Zahns als ausgewählten Bereich, die
- Fig. 4: den Zahn aus Fig. 1 in einer Ansicht in bukkaler Richtung mit der Oberfläche des vorderen Bereiches des Zahns oberhalb der Äquatorlinie als ausgewählten Bereich, die
- Fig. 5: den Zahn aus Fig. 1 in einer Ansicht mesial/von oben mit der Okklusalfläche als ausgewählten Bereich, die
- Fig. 6: den Zahn aus Fig. 1 in einer Schrägansicht aus bukkaler Richtung mit einem Höcker als ausgewählten Bereich, die
- Fig. 7: ein Restaurationskörper in Form eines Frontzahnes in einer Schrägansicht aus lingual/distaler Richtung mit einer Hälfte des Zahnes als ausgewählten Bereich, die
- Fig. 8: den Zahn aus Fig. 7 mit dem Okklusalbereich oberhalb der Labiolinguallinie als ausgewählten Bereich und die
- Fig. 9: den prinzipiellen Aufbau der Vorrichtung.

### Ausführungsbeispiel

In einem CAD-System werden in einer 3D-Darstellung der Restauration dentalspezifische Linien und Punkte angezeigt. Durch Anklicken einzelner Elemente erfolgt die Auswahl eines beispielsweise mit Konstruktionswerkzeugen zu bearbeitenden Bereiches, wobei unmittelbar in der zu bearbeitenden Darstellung gearbeitet wird. Als Konstruktionswerkzeug kann insbesondere eine Formveränderung zur Anwendung kommen, die die Oberfläche von den Bereichsgrenzen ausgehend bis zum Punkt der stärksten Änderung verändert.

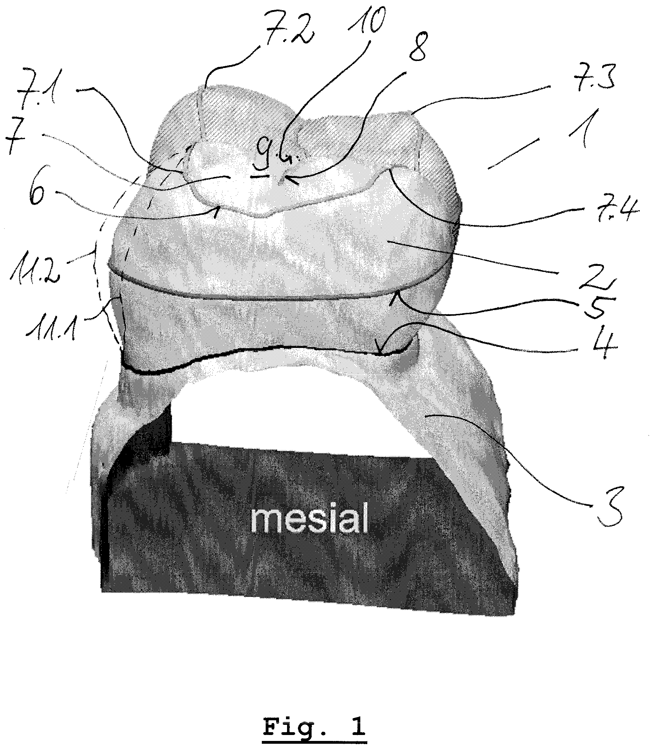

In Fig. 1 ist ein zu erstellender Restaurationskörper in Form eines Backenzahns 1 aus mesialer Richtung gesehen dargestellt, wobei ein der vorderen Hälfte des Zahn entsprechender Bereich 2 ausgewählt ist. In der 3D-Darstellung des Zahnes 1 ist der nichtausgewählte Bereich schraffiert dargestellt, darüber hinaus sind verschiedene Linien und Punkte angezeigt, die nachfolgend erläutert werden.

Ausgehend vom unteren, zervikalen Ende des Zahns 1 welches im Bereich der Gingiva 3 angeordnet ist, ist eine erste Linie in Form des Präparationsrandes 4 dargestellt. Der Präparationsrand 4 stellt die Grenze zwischen der originalen Zahnoberfläche im nicht präparierten Zustand und dem präparierten Teil des Zahns dar.

Im Bereich des größten Umfanges des Zahns 1 ist die Äquatorlinie 5 als eine von mehreren dentalspezifischen Linien eingezeichnet. Weiter fortschreitend zur Oberseite des Zahns 1 hin ist eine Randleiste 6 eingezeichnet, welche über Zahnhöcker 7 verläuft, die durch dentalspezifische Punkte 7.1 bis 7.4 besonders gekennzeichnet sind.

Eine weitere dentalspezifische Linie ist eine Fissur 8, die auf der Oberfläche des Zahns 1 innerhalb einer von der Randleiste 6 begrenzten Okklusalfläche 9 verläuft.

Eine weitere Bereichsgrenze ist eine Trennlinie 10 zwischen der vorderen, mesialen Hälfte und der hinteren, distalen Hälfte des Zahns 1, welche dem Verlauf einer Seitenfissur entsprechen kann, aber auch einfach eine Art geometrische Mitteilung darstellen kann. Der Verlauf dieser Linie ist in der hier beschriebenen Darstellung dentalmedizinisch von untergeordneter Bedeutung.

Der Bereich 2 wird durch Auswahl des Präparationsrandes 4, beispielsweise durch Anklicken mit einem Mauszeiger ausgewählt und erstreckt sich vom Präparationsrand 4 bis zur Mitte der Okklusalfläche 9, die durch Verlauf der Linie 10 angedeutet ist.

Es versteht sich, dass anstelle des Anklickens des Präparationsrandes 4 auch vorgesehen werden kann, bei Anklicken der Bereiche zwischen den vorstehend beschriebenen Linien oder Punkten eine Auswahl herbeizuführen. Beispielsweise kann durch Anklicken des Bereichs zwischen dem Präparationsrand 4 und der Äquatorlinie 5 die in Fig. 1 dargestellte Zahnhälfte ausgewählt werden. Ein Anklicken anderer Bereiche in der Darstellung führt zu einem anders begrenzten ausgewählten Bereich.

Mit einem Bearbeitungswerkzeug lässt sich der gesamte Bereich 2 bearbeiten, in dem beispielsweise über Tastatureingaben der Bereich breiter oder schmäler, höher oder niedriger oder vorspringend oder zurückgezogen wird. Der Bereich lässt sich demnach in allen sechs Raumrichtungen verändern, angedeutet durch die Linien 11.1, 11.2 nach Verjüngen bzw. Verbreitern der Zahnbreite.

Gleiches gilt selbstverständlich für die Höhe des Zahn bzw. die räumliche Ausdehnung zum Betrachter hin. Dabei kann sich auch der Verlauf der Fissur 8 ändern.

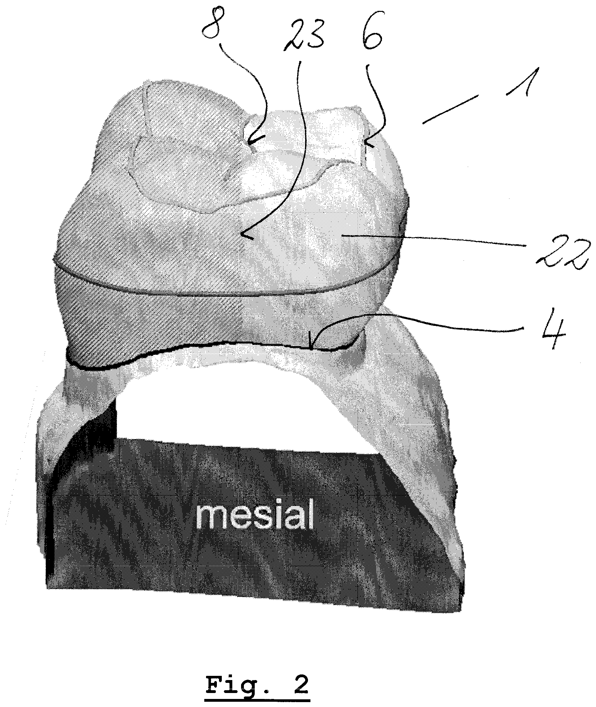

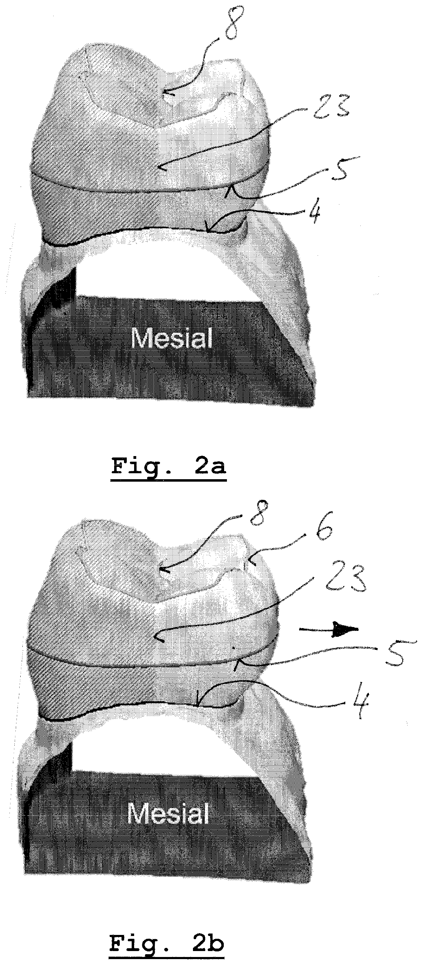

In Fig. 2 ist der Zahn 1 aus der selben Richtung wie in Fig. 1 dargestellt, allerdings ist nun die rechte, linguale Hälfte des Zahn der ausgewählte Bereich 22. In dieser Darstellung ist besonders gut zu erkennen, dass eine Grenzlinie 23 des ausgewählten Bereichs 22 keine dentaltypische Konstruktionslinie ist, sondern lediglich eine Hilfslinie zur Abgrenzung des Bereiches 22. Die Linie 23 verläuft etwa in der Mitte des Zahns 1 und damit in etwa im Bereich der Seitenfissuren. Selbstverständlich kann auch die Seitenfissur selbst als dentalspezifische Linie für die Begrenzung des Bereichs 22 herangezogen werden, wenn sie aus den Messdaten oder in sonstiger Weise bestimmt wurde.

Wird der ausgewählte Bereich 22 mit dem Werkzeug für die CAD-Konstruktion bearbeitet, so ist die Präparationsgrenze 4 und die Hilfslinie 23 unveränderlich.

Fig. 2a,b ist der Zahn aus Fig. 2 mit der rechten Hälfte des Zahn als ausgewählten Bereich in einer Gegenüberstellung vor einer Veränderung (Fig. 2a) und nach einer Veränderung (Fig. 2b) gezeigt, wobei die Änderung in Pfeilrichtung erfolgte. Dabei hat sich der Verlauf des Präparationsrandes 4 und der Linie 23 nicht verändert, wohingegen die Äquatorlinie 5 und die Randleiste 6 sowie die Fissur ihre Lage verändert haben in Richtung des Pfeils.

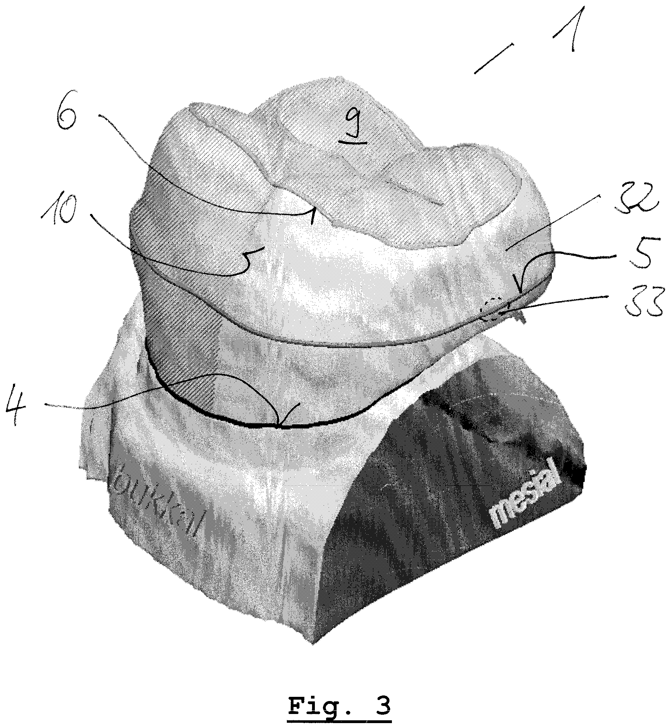

In Fig. 3 ist der Zahn 1 in einer Schrägansicht dargestellt, wobei der interessierende ausgewählte Bereich 32 die Seitenfläche der vorderen Hälfte des Zahns 1 aus Fig. 1 darstellt. Die Okklusionsfläche 9 ist daher im Unterschied zu Fig. 1 nicht mehr Teil des ausgewählten Bereichs 32. Der ausgewählte Bereich 32 wird begrenzt durch den Präparationsrand 4, die Hilfslinie 10 und durch die Randleiste 6.

Wird nun mit dem Konstruktionswerkzeug der Bereich 32 bearbeitet, so dehnt sich beispielsweise lediglich die Seitenfläche in mesialer Richtung weiter aus, ohne dass sich die Randleiste 6, die Hilfslinie 10 oder der Präparationsrand 4 verändert.

Die Änderungen des Verlaufs der Oberfläche erfolgen dabei so, dass die Änderung ausgehend von der Änderung "0" an den vorstehend beschriebenen Grenzen des Bereichs 32 bis zu dem Punkt der stärksten Änderung, angedeutet durch den gestrichelten Kreis 33 bei Veränderung in mesialer Richtung stetig verläuft, so dass die Oberfläche mit Ausnahme des Randbereiches in dem gesamten Bereich 32 verändert wird. Der Bereich 32 wird beispielsweise durch Anklicken der Äquatorlinie 5 ausgewählt.

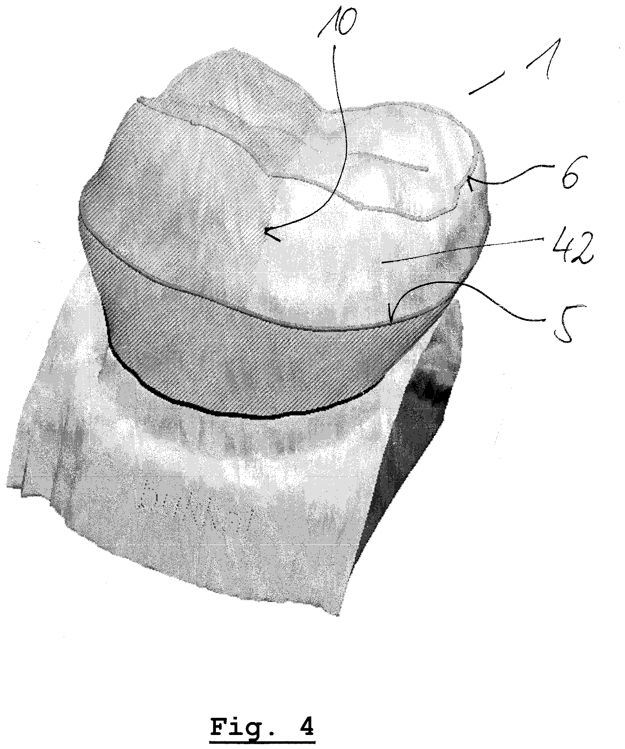

In Fig. 4 ist der aus Fig. 1 bekannte Zahn 1 in einer Ansicht in bukkaler Richtung dargestellt, wobei der ausgewählte Bereich 42 nur die Oberfläche des vorderen Bereichs des Zahn oberhalb der Äquatorlinie 5 ist. Die Grenzen des ausgewählten Bereichs 42 werden demnach durch die Äquatorlinie 5 und die Hilfslinie 10 gegeben. Die Hilfslinie 10 verläuft in etwa im Bereich der Seitenfissuren, wobei hier jedoch der genaue Verlauf nicht von entscheidender Bedeutung ist. Die Auswahl des Bereichs 42 erfolgt beispielsweise durch Anklicken des rechten Teils der Randleiste 6.

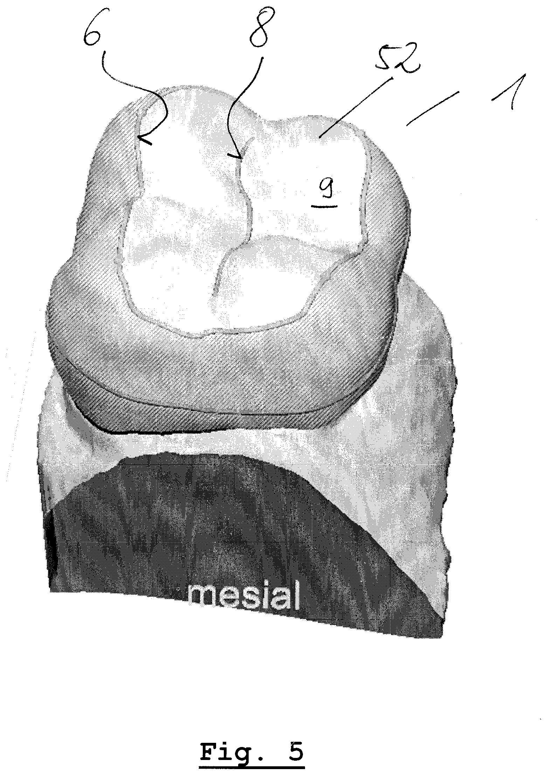

In Fig. 5 ist der Zahn 1 aus Fig. 1 in einer Ansicht mesial schräg von oben dargestellt, wobei als ausgewählter Bereich 52 die Okklusalfläche 9 gezeigt ist. Die Okklusalfläche 9 wird begrenzt von der Randleiste 6. Änderungen wirken sich nur auf die Lage, nicht aber auf den Verlauf der Fissur 8 aus. Dies bedeutet, dass sich die räumliche Beziehung der die Fissur 8 bildenden Punkte untereinander nicht verändert, insbesondere auch dass die Linie nicht gestreckt oder verbogen wird. Die gesamte Linie wird bei Änderung der Okklusalfläche 9 beispielsweise angehoben oder abgesenkt.

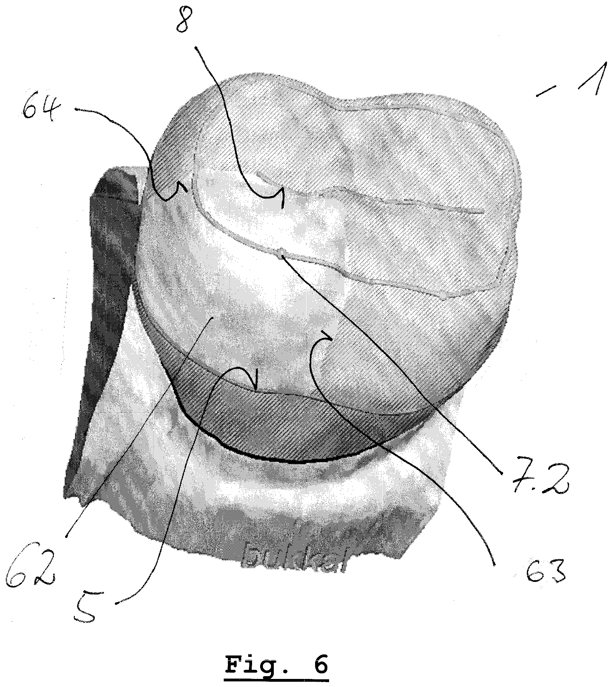

In Fig. 6 ist der aus Fig. 1 bekannte Zahn in einer Schrägansicht aus bukkaler Richtung gezeigt, wobei als ausgewählter Bereich 62 ein Höcker, gekennzeichnet durch Punkt 7.2, dargestellt ist. Der ausgewählte Bereich 62 mit dem Höcker wird nach unten durch die Äquatorlinie 5 und seitlich durch die Höckergrenzen 63, 64 begrenzt. Die Höckergrenze 64 verläuft entlang der Fissur 8 und einer Verlängerung davon und geht in die Seitenfissur über, die Höckergrenze 63 besteht aus der Seitenfissur und deren Verlängerung.

Der Höckerpunkt 7.2 lässt sich nun zusammen mit der restlichen Oberfläche in allen sechs Raumachsen bearbeiten. So kann er weiter herausgehoben oder abgesenkt werden, in distale oder mesiale Richtung verschoben werden, ebenso in bukkale oder linguale Richtung. Auch hier sind die Grenzen des Bereichs 62 unveränderlich. Die Auswahl des Bereich 62 erfolgt beispielsweise durch Anklicken des Punktes 7.2.

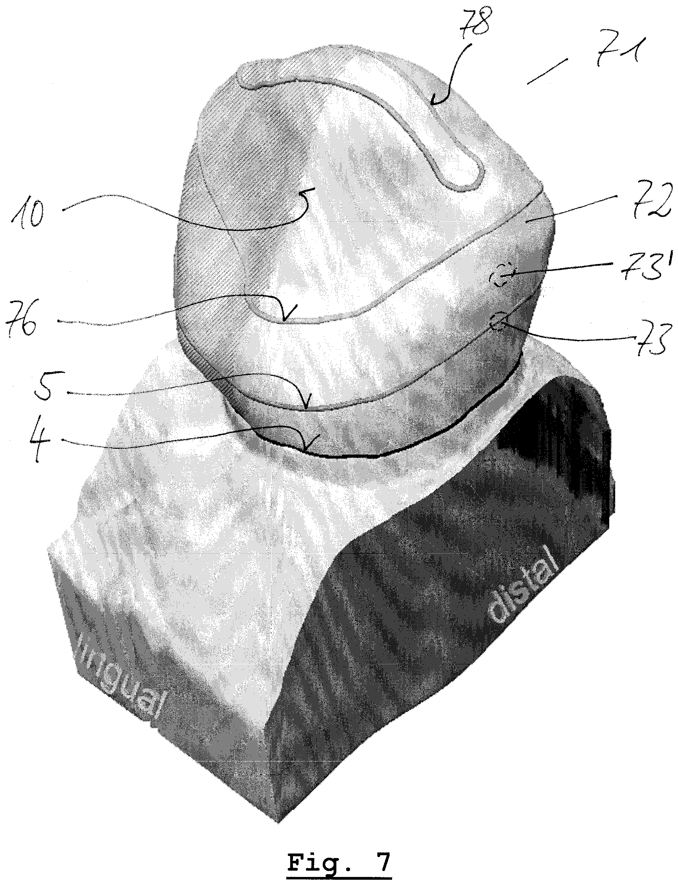

In Fig. 7 ist ein Frontzahn 71 in einer Schrägansicht aus lingual/distaler Richtung gezeigt. Als ausgewählter Bereich 72 ist eine Hälfte des Zahns 71 dargestellt. Ausgehend vom Präparationsrand 4 findet sich wieder die Äquatorlinie 5 und die Labiolinguallinie 76 anstelle der Randleiste. An der Spitze des Zahns 71 ist die Schneidekante 78 gezeigt, welche den Bereich der Inzisalkante umschließt.

Darüber hinaus ist die Hilfslinie 10 dargestellt, welche den Zahn 71 in zwei Hälften teilt. Der ausgewählte Bereich 72 wird demnach begrenzt durch die Präparationslinie 4 und die Hilfslinie 76 und die Oberfläche des Bereichs 72 kann in allen sechs Raumrichtungen verändert werden, wobei an den Bereichsgrenzen keine Veränderung stattfindet und wiederum eine stetige Veränderung bis zum Punkt der größten Veränderung, angedeutet durch den gestrichelten Kreis 73 bei Änderung in distaler Richtung erfolgt.

Der Bereich 73 der größten Änderung liegt auf der dentalspezifischen Linie, nämlich auf der Äquatorlinie 5. Selbst wenn andere Bereiche 73' vorhanden sind, die zu einem Zentrum oder einer Mittelachse einen größeren Abstand haben, werden diese durch die Anwendung der Konstruktionswerkzeuge weniger stark verformt. Dies gilt auch für die anderen Ausführungsbeispiele.

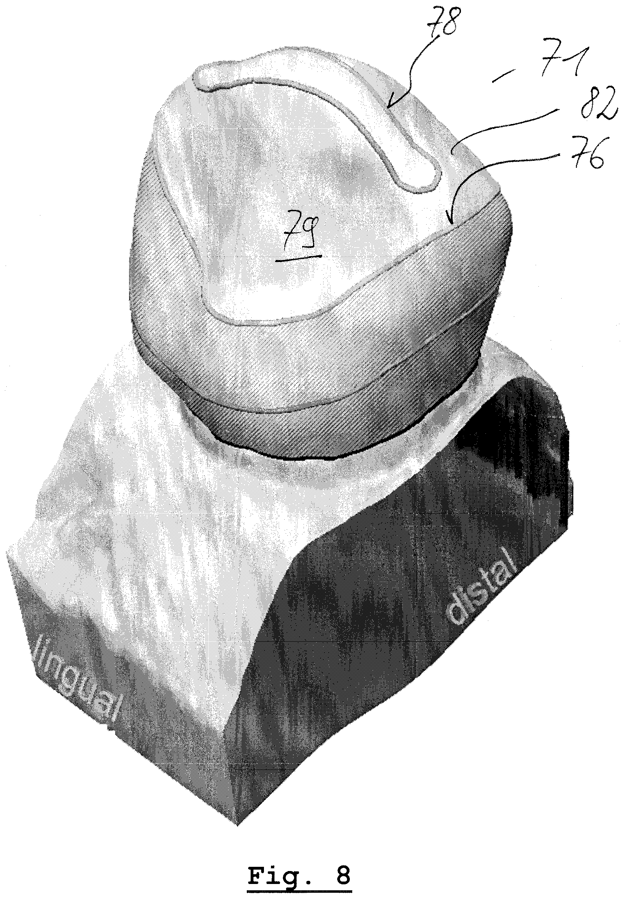

In Fig. 8 ist der aus Fig. 7 bekannte Zahn 71 in der selben Ansicht dargestellt, allerdings ist nun der Okklusalbereich 79 oberhalb der Labiolinguallinie 76 ausgewählt.

Änderungen in diesem Bereich 72 wirken sich auch auf die Schneidekante 78 aus, wie dies bereits bei der Veränderung des Okklusalbereichs gemäß Fig. 5 erläutert wurde. Die Lage der Schneidkante 78 kann verändert werden, nicht jedoch deren Verlauf als solcher.

Grundsätzlich gilt, dass die dentalspezifischen Konstruktionslinien vom CAD-System anhand des vorhandenen 3D-Datensatzes vorgeschlagen werden und von Hand noch angepasst werden können unter Berücksichtigung dentalspezifischer Problemstellungen. Dies gilt auch für dentalspezifische Punkte wie für die Positionen der Höckerspitzen.

Jede Linie 4, 5, 6 wird in vier Teile unterteilt, die jeweils die mesiale, distale, bukkale bzw. linguale Seite des Zahns repräsentieren. Durch Auswahl eines Teilbereiches der Linie, beispielsweise durch Anklicken, wird die Hälfte des Zahnes festgelegt, in der der ausgewählte Bereich liegt.

Je nachdem, welche der Linien ausgewählt wird, wird ein entsprechender Bereich des Zahns ausgewählt. Bei Auswahl des Präparationsrand 4 geht der ausgewählte Bereich 2; 22 vom Präparationsrand 4 bis Mitte der Okklusalfläche 9. Bei Auswahl der Äquatorlinie 5 liegt der ausgewählte Bereich 32 zwischen dem Präparationsrand und der Randleiste 6. Bei Auswahl der Randleiste 6 geht der Bereich 42 von der Äquatorlinie 5 bis zur Mitte der Okklusalfläche Bei Auswahl der Fissur 8 wird die gesamte Okklusalfläche 9 als Bereich 52 ausgewählt, der von der Randleiste 6 begrenzt ist.

Wie bei der Beschreibung der Fig. 1 bereits angemerkt, kann auch durch anklicken der dargestellten Oberfläche des Zahns außerhalb der Linien oder der dental spezifischen Punkte ein Bereich auswählbar sein. Ein anklicken eines Zahnhöckers im Bereich seiner höchsten Erhebung kann zur Auswahl des Zahnhöckers führen. Ein anklicken eines Bereichs zwischen zwei Höckern kann zur Auswahl der gesamten Okklusalfläche führen. Ein anklicken der Oberfläche zwischen der Äquatorlinie und der Randleiste kann zur Auswahl gemäß Fig. 3 oder Fig. 4 führen, je nachdem, ob man beispielsweise näher an der Äquatorlinie oder näher an der Randleiste die Bereichsauswahl vornimmt. Auch hier gilt wieder, das die Oberfläche in die mesiale, distale, bukkale bzw. linguale Seite des Zahnes eingeteilt ist, sodass der jeweils interessierende Bereich eindeutig festgelegt ist.

Für den Frontzahn gilt entsprechend, das zur Auswahl des in Fig. 7 dargestellten Bereichs beispielsweise ein Bereich unterhalb der Laviolinguallinie angeklickt wird, was zur Auswahl gemäß Fig. 8 führt. Wird hingegen ein Bereich unterhalb der Laviolinguallinie ausgewählt, erhält man eine Auswahl gemäß Fig. 7.

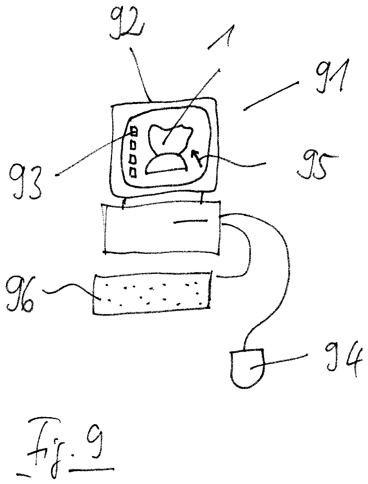

In Fig. 9 ist der prinzipiellen Aufbau der Vorrichtung gezeigt. Die Vorrichtung umfasst ein CAD-System 91 mit einer Anzeigeeinheit 92 zur Darstellung des Restaurationskörpers 1 und zur Auswahl eines durch Symbole dargestellten Konstruktionswerkzeugs 93. Weiterhin ist eine Eingabeeinheit in Form einer Computermaus 94 vorhanden, über die ein auf der Anzeigeeinheit 92 angezeigter Zeiger 95 steuerbar ist. Zusätzlich oder alternativ dazu ist eine Tastatur 96 vorgesehen, mit der Eingaben zur Steuerung des CAD-Systems erfolgen.

## Patentansprüche

1. Vorrichtung zur Auswahl eines Bereichs (2; 22; 32; 42; 52; 62; 72; 82) eines in einer 3D-Darstellung dargestellten dentalen Restaurationskörpers (1; 71), umfassend ein CAD-System (91) mit einer Anzeigeeinheit (92) zur Darstellung des Restaurationskörpers (1; 71) und mit einer Eingabeeinheit (94), über die ein auf der Anzeigeeinheit (92) angezeigter Zeiger (95) steuerbar ist, wobei zumindest Teile der Bereichsgrenze als dentalspezifische Linien (5, 6, 8) ausgebildet sind, **dadurch gekennzeichnet, dass** die Vorrichtung so gestaltet ist, dass der Bereich (2; 22; 32; 42; 52; 62; 72; 82) durch Auswahl in der 3D-Darstellung angezeigter dentalspezifischer Punkte (7.1-7.4) festlegbar ist und dass durch Auswahl eines dentalspezifischen Punktes (7.1-7.4) für die Position der Höckerspitze der Bereich (62) des entsprechenden Höckers (7) bis zu seinen Höckergrenzen auswählbar ist und dass die untere Grenze des Bereichs (62) durch die Äquatorlinie (5) gebildet ist, wobei die Auswahl durch Anklicken des dentalspezifischen Punktes mittels der Eingabeeinheit (94) über den Zeiger (95) erfolgt.

2. Vorrichtung nach Anspruch 1, **dadurch gekennzeichnet, dass** der ausgewählte Bereich (2; 22; 32; 42; 52; 62; 72; 82) unterscheidbar dargestellt ist.

3. Vorrichtung nach Anspruch 1 oder 2, **dadurch gekennzeichnet, dass** Auswerte- und/oder Vergleichsmittel für geometrische Daten des ausgewählte Bereichs (2; 22; 32; 42; 52; 62; 72; 82) vorgesehen sind.

4. Vorrichtung nach einem der Ansprüche 1 bis 3, **dadurch gekennzeichnet, dass** der ausgewählte Bereich (2; 22; 32; 42; 52; 62; 72; 82) mit Hilfe eines Konstruktionswerkzeugs eines CAD-Systems bearbeitbar ist.

5. Vorrichtung nach Anspruch 4, **dadurch gekennzeichnet, dass** das Werkzeug zur Veränderung des Bereiches (2; 22; 32; 42; 52; 62; 72; 82) die Bereichsgrenzen unverändert lässt, wobei zu dem Punkt (33; 73) der stärksten Änderung hin ein stetiger Verlauf der Änderung vorgesehen ist.

6. Vorrichtung nach Anspruch 5, **dadurch gekennzeichnet, dass** der Punkt (33; 73) der stärksten Änderung auf einem dentalspezifischen Punkt (7.1-7.4) liegt.

7. Verfahren zur Auswahl eines Bereichs (2; 22; 32; 42; 52; 62; 72; 82) eines in einer 3D-Darstellung dargestellten dentalen Restaurationskörpers (1; 71) umfassend ein CAD-System (91) mit einer Anzeigeeinheit (92) zur Darstellung des Restaurationskörpers (1; 71) und mit einer Eingabeeinheit (94), über die ein auf der Anzeigeeinheit (92) angezeigter Zeiger (95) steuerbar ist, wobei dem ausgewählten Bereich Bereichsgrenzen zugeordnet werden, die zumindest zum Teil als dentalspezifische Linien (5, 6, 8) ausgebildet sind, **dadurch gekennzeichnet, dass** durch Auswahl eines dentalspezifischen Punktes für die Position der Höckerspitze der Bereich (62) der entsprechenden Höcker (7) bis zu seinen Höckergrenzen ausgewählt wird und dass die untere Grenze des Bereichs (62) durch die Äquatorlinie (5) gebildet ist, wobei die Auswahl durch Anklicken des dentalspezifischen Punktes mittels der Eingabeeinheit (94) über den Zeiger (95) erfolgt.

## Claims

1. A device for selecting an area (2; 22; 32; 42; 52; 62; 72; 82) of a dental restoration body (1; 71), which is depicted in a 3D representation, comprising a CAD system (91) having a display unit (92) for representing the restoration body (1; 71) and having an input unit (94) by means of which a pointer (95) displayed on the display unit (92) can be controlled, at least parts of the area boundary being in the form of dental-specific lines (5, 6, 8), **characterised in that** the device is designed in such a way that the range (2; 22; 32; 42; 52; 62; 72; 82) can be set by selection in the 3D representation of displayed dental-specific points (7.1-7.4) and that, by selection of a dental-specific point (7.1-7.4) for the position of the cusp tip, the area (62) of the relevant cusp (7) can be selected up to the boundaries of its cusp, and that the lower limit of the area (62) is formed by the equator line (5), the selection being made by clicking on the dental-specific point by means of the input unit (94) via the pointer (95).

2. The device according to claim 1, **characterised in that** the selected area (2; 22; 32; 42; 52; 62; 72; 82) is distinguishably represented.

3. The device according to claim 1 or 2, **characterised in that** means of evaluation and/or comparison for geometric data of the selected area (2; 22; 32; 42; 52; 62; 72; 82) are provided.

4. The device according to any of claims 1 to 3, **characterised in that** the selected area (2; 22; 32; 42; 52; 62; 72; 82) can be edited with the aid of a construction tool of a CAD system.

5. The device according to claim 4, **characterised in that** the tool for changing the area (2; 22; 32; 42; 52; 62; 72; 82) leaves the boundaries of the area unchanged, wherein a constant track of the change is provided at the point (33; 73) of the greatest change.

6. The device according to claim 5, **characterised in that** the point (33; 73) of the greatest change is located on a dental-specific point (7.1-7.4).

7. A method for selecting an area (2; 22; 32; 42; 52; 62; 72; 82) of a dental restoration body (1; 71), which is depicted in a 3D representation, comprising a CAD system (91) having a display unit (92) for representing the restoration body (1; 71) and having an input unit (94) by means of which a pointer (95) displayed on the display unit (92) can be controlled, area boundaries being assigned to the selected area which are at least partially in the form of dental-specific lines (5, 6, 8), **characterised in that,** by selection of a dental-specific point for the position of the cusp tip, the area (62) of the corresponding cusp (7) is selected up to its cusp boundaries and that the lower limit of the area (62) is formed by the equator line (5), wherein the selection is made by clicking on the dental-specific point by means of the input unit (94) via the pointer (95).

## Revendications

1. Dispositif permettant de sélectionner une zone (2 ; 22 ; 32 ; 42 ; 52 ; 62 ; 72 ; 82) d'un corps de restauration dentaire (1 ; 71) figurant sur une représentation tridimensionnelle, comprenant un système de CAO (91) comportant une unité d'affichage (92) pour afficher le corps de restauration (1 ; 71) et une unité d'entrée (94) par laquelle peut être commandé un pointeur (95) affiché sur l'unité d'affichage (92), au moins des parties de la limite de zone étant formées comme des lignes dentaires spécifiques (5, 6, 8), **caractérisé en ce que** le dispositif est conçu de telle sorte que la zone (2 ; 22 ; 32 ; 42 ; 52 ; 62 ; 72 ; 82) puisse être déterminée par sélection des points dentaires spécifiques indiqués (7.1-7.4) dans la représentation tridimensionnelle et **en ce que** par sélection d'un point dentaire spécifique (7.1-7.4) pour la position de la pointe de la cuspide, la zone (62) de la cuspide correspondante (7) peut être sélectionnée jusqu'à ses limites de cuspides et **en ce que** la limite inférieure de la zone (62) est définie par la ligne équatoriale (5), la sélection étant effectuée en cliquant sur le point spécifique dentaire à l'aide de l'unité d'entrée (94) par l'intermédiaire du pointeur (95).

2. Dispositif selon la revendication 1, **caractérisé en ce que** la zone sélectionnée (2 ; 22 ; 32 ; 42 ; 52 ; 62 ; 72 ; 82) est représentée distinctement.

3. Dispositif selon la revendication 1 ou 2, **caractérisé en ce qu'**on prévoit des moyens d'évaluation et/ou de comparaison de données géométriques de la zone sélectionnée (2 ; 22 ; 32 ; 42 ; 52 ; 62 ; 72 ; 82).

4. Dispositif selon l'une des revendications 1 à 3, **caractérisé en ce que** la zone sélectionnée (2 ; 22 ; 32 ; 42 ; 52 ; 62 ; 72 ; 82) peut être traitée à l'aide d'un outil de construction d'un système de CAO.

5. Dispositif selon la revendication 4, **caractérisé en ce que** l'outil pour modifier la zone (2 ; 22 ; 32 ; 42 ; 52 ; 62 ; 72 ; 82) laisse les limites de la plage inchangées, une évolution constante étant prévue en direction du point (33 ; 73) présentant le changement le plus important.

6. Dispositif selon la revendication 5, **caractérisé en ce que** le point (33 ; 73) présentant le changement le plus important se situe sur un point dentaire spécifique (7.1-7.4).

7. Procédé permettant de sélectionner une zone (2 ; 22 ; 32 ; 42 ; 52 ; 62 ; 72 ; 82) d'un corps de restauration dentaire (1 ; 71) figurant sur une représentation tridimensionnelle, comprenant un système de CAO (91) comportant une unité d'affichage (92) pour afficher le corps de restauration (1 ; 71) et une unité d'entrée (94) par laquelle peut être commandé un pointeur (95) affiché sur l'unité d'affichage (92), des parties de limite étant associées à la zone sélectionnée, qui sont formées, au moins en partie, comme des lignes dentaires spécifiques (5, 6, 8), **caractérisé en ce que** par sélection d'un point dentaire spécifique pour la position de la pointe de la cuspide, la zone (62) de la cuspide correspondante (7) peut être sélectionnée jusqu'à ses limites de cuspides et **en ce que** la limite inférieure de la zone (62) est définie par la ligne équatoriale (5), la sélection étant effectuée en cliquant sur le point spécifique dentaire à l'aide de l'unité d'entrée (94) par l'intermédiaire du pointeur (95).
